(19) Europäisches Patentamt
European Patent Office
Office européen des brevets

(11) **EP 3 682 864 A1**

(12) **EUROPEAN PATENT APPLICATION**
published in accordance with Art. 153(4) EPC

(43) Date of publication:
**22.07.2020 Bulletin 2020/30**

(21) Application number: **18808128.5**

(22) Date of filing: **12.09.2018**

(51) Int Cl.:
*A61K 8/44* (2006.01)  *A61Q 5/00* (2006.01)

(86) International application number:
**PCT/RU2018/050111**

(87) International publication number:
**WO 2019/054906 (21.03.2019 Gazette 2019/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2017 RU 2017133704**

(71) Applicant: **Obshchestvo S Ogranichennoj Otvetstvennostyu "Yunikosmetik" St.Petersburg 195273 (RU)**

(72) Inventor: **CHISTYAKOVA, Olga Ivanovna St.Petersburg 193312 (RU)**

(74) Representative: **Viering, Jentschura & Partner mbB**
**Patent- und Rechtsanwälte**
**Hamborner Straße 53**
**40472 Düsseldorf (DE)**

(54) **USE OF A DIAMINE TO IMPROVE THE MECHANICAL STRENGTH OF HAIR, AND HAIR CARE AGENT CONTAINING SAME**

(57) The present invention relates to use of at least one $\alpha,\omega$-diamine of the general formula $H_2N\text{-}CH_2\text{-}(CH_2)_n\text{-}CH_2\text{-}NH_2$ or its salt, where n = 0, 1, 2, 3, 4 or 5, to improve the hair mechanical strength, and to the hair care product containing it. The invention improves the hair mechanical strength.

EP 3 682 864 A1

**Description**

TECHNICAL FIELD

The invention relates to the cosmetic industry and can be used to improve hair mechanical strength.

PRIOR ART

Human hair is identical to animal hair to a large extent - the main "construction material" in both cases is the keratin protein. Keratin consists of 19 amino acids and takes up 90% of hair mass. Each hair consists of 3 layers: the outer layer or the cuticle is represented by elongated keratin scales that overlap each other like chain mail scales, and act as an external protective layer. Under the cuticle there is the second layer - cortex, consisting of elongated cells, giving the hair strength and suppleness. The hair center consists of a medullary substance which includes soft keratin cells and air spaces. The hair follicle is located under the skin. The shape of follicle determines the hair type: straight hair grows from round follicle, wavy - from oval follicle, curly - from kidney-shaped follicle. The shape of follicle also determines structure of amino acid chains: when amino acid chains do not have additional bonds, the hair becomes straight, if more bonds are formed between acids, the hair becomes wavy. From the biochemistry point of view, disulfide and hydrogen bonds, as well as ionic interaction of functional groups of keratin side chains, play the role of such additional bonds.

Change of natural hair color has been practiced since ancient times, and natural dyes have been used for this purpose for many centuries. Only from the end of the XIX century, as the chemistry of synthetic dyes developed, they started to be used not only for dyeing fabrics, but also for dyeing hair. The background for this were definitive similarities between human hair and animal hair. As a consequence, such technical classes of dyes as acidic, basic (cationic), active, dispersed and others that were used for wool dyeing, were used for hair dyeing. Subsequently, these dyes were called direct dyes. However, as it turned out, the colors obtained with these dyes were very unstable and could only be used to temporarily dye the hair. This is quite explainable, since large dye molecules can not penetrate the hair interior, and they are fixed only on the surface due to Van der Waals forces, London forces, and hydrogen bonds.

Hair dyeing formulations, which are water solutions of dyes [US7198651B2 published on April 03, 2007 by FUJI PHOTO FILM CO., LTD; US7241318B2 published on July 10, 2007 by CREATE CO.], have certain disadvantages when applying to hair: the scalp is intensively colored and the dye flows into the face, while the hair coloring itself is not intense enough. In order to overcome such difficulties during application, it is proposed to thicken the hair coloring compositions to some extent, producing gels that are better retained on the hair, but do not interfere with dye transition to the hair keratin [US6740129B2 published on May 25, 2004 by HENKEL LION COSMETICS CO. LTD]. There are creamy formulations that are even more comfortable to use, but they more strongly inhibit dye transition to the hair, which requires increasing the coloring time. The creamy consistency is obtained by introducing various fillers: granulated cellulose [US7204856B2 published on April 17, 2007 by HENKEL KGAA]; thickening polymers of anionic, cationic and non-ionic types [US7300470B2 published on November 27, 2007 by L'OREAL S.A.]. Thickening emulsions are widely used, for example, formulations containing high-molecular alcohols C12-C20, glycerin monostearate, potassium salts of fatty acids [US7204859B2 published on May 25, 2010 by L'OREAL S.A.]. But creamy emulsions have one more drawback - due to their high density, it is not always possible to achieve even application and hair coloring in general. For that reason, foam hair dyeing formulations are produced, which are easy and convenient to apply, they properly penetrate the hair, offering uniform coloring.

The first foam hair dyeing formulations were developed on the basis of direct dyes placed in conventional tubes [WO2009077302A2 published on June 25, 2009 by HENKEL AG & CO KGAA], and then on the basis of two-phase systems [DE102006055436A1 published on October 11, 2007 by HENKEL KGAA]. Cationic polymeric materials were introduced in dye formulations in order to provide foaming thereof.

In most cases it is suggested to use oxidation dyes as dyes for deep and long lasting (persistent) hair coloring [FR2870733B1 published on July 07, 2006 by L'OREAL SOCIETE ANONYME; US7044986B2 published on May 16, 2006 by KAO CORPORATION; US7056497B2 published on June 06, 2006 by PROCTER & GAMBLE]. This is determined by the following requirements to hair dyes: 1) dyeing should be performed in a short time at room temperature and should not cause damage to the hair core or scalp; 2) dyes should give the hair a natural color and cause minimal coloring of the scalp; 3) short dyeing time requires the use of formulations with high dye concentrations; 4) diffusion of the dye into human hair is much slower than diffusion into keratin of wool, therefore small dyes of low molecular weight are required. Only oxidation dyes meet these requirements.

Direct dyes - nitro, acidic, basic (cationic), active, dispersed - are added to dye formulations to provide the desired shade, and they are generally retained on the hair surface due to formation of hydrogen bonds, Van der Waals forces and London forces, without deep penetration due to large size of molecules and low diffusion rate - as a result, they wash off quickly.

The revolutionary step in hair dyeing was the proposal to use not the ready-made dyes, but intermediates for their production: aromatic diamines and amino phenols. Molecules of these compounds are small, easily penetrate inside the hair and, under action of oxidizing agent, they transform there into colored indamines, indanilines and indophenols, which then di- and trimerize to form heterocyclic pigments with high molecular weight that are unable to exit the hair, providing high coloring strength. These intermediates were called oxidation dyes. By analogy with the photographic process, these compounds are also called developers. These include aromatic o- and p-diamines of benzene and naphthalene series, o- and p-amino phenols and their derivatives, analogous derivatives of pyridine, pyrimidine and other heterocyclic series.

When these compounds are oxidized, for example, with hydrogen peroxide, persulphates, perborates, bichromate and chlorate of potassium or sodium, they transform, as already mentioned, to quinone imines, and then to indamines or indanilines. Further oxidation leads to formation of phenazine structures containing several hetero-rings. As a mixture of many products is formed as a result of these reactions, the hair is dyed into soft, blurry shades, completely imitating natural colors: darkest blond, chestnut, maroon.

Developers, or color bases, are usually used in a mixes with so-called color components. These are m-diamines, m-amino phenols, phenols, naphthols, pyrazolones, β-diketones and their derivatives. These compounds do not provide coloring by themselves, but when reacting with the developer they expand the color range of dyeing formulations, for example, p-phenylenediamine with m-toluylenediamine forms a red phenazine colorant, and with 2,4-diaminoanisole it forms violet-blue indamine:
In order to ensure effective penetration of both oxidation dyes and oxidants into the hair, the coloring process is usually performed in an alkaline medium that destroys the outer hair protective layer and loosens the inner hair layers. Of course, this causes irreparable hair damage: the hair lose the gloss, strength, become fragile, brittle, thin; the hair experience a significant decrease in tensile strength. This is especially noticeable with continual coloring.

Therefore, along with improvement of coloring formulations, production of hair conditioners intended to at least mitigate harmful effects of dyeing is intensively developed in the last decade.

The conditioners have been used for many centuries. Since ancient times people have treated hair with oils and fats to give them gloss and protect them from environmental factors (sun rays). Many of these conditioning additives are used until now, for example, vegetable and essential oils, beeswax. Modern conditioners include not only natural components, but also various chemically synthesized substances. These are different silicones and carbon polymers, which have thermal protection, smoothing and light-reflecting properties, facilitate hair combing and prevent their tangling. At the same time, almost all these compounds have a very long polymer chain, due to which they attach to the hair from outside, so frequent use or wrong choice of a conditioner can result in oily hair, that will clot and quickly get dirty. This size, moreover, prevents penetration of active molecules into the hair.

There is a biphasic formulation for hair dyeing [RU2493814C1 published on September 27, 2013 by UNICOSMETIC Co. Ltd.], containing a mixture of quaternium-70 (N,N-dimethyl-3-((1-oxooctadecyl)amino)-N-(2-oxo-2-(tetradecenyloxy)ethyl)-1-propanamin chloride), hydrolyzed keratin, avocado oil and dimethicone (polydimethylsiloxane) as the conditioning agent.

There is a biphasic formulation for hair dyeing [RU2541811C1 published on February 20, 2015 by UNICOSMETIC Co. Ltd.], containing a mixture of cetearylmethicone and dimethiconol as the conditioning agent.

There are biphasic formulations for hair dyeing [RU2572708C2 published on January 20, 2016, RU2602690C1 published on November 20, 2016 both by UNICOSMETIC Co. Ltd.], containing PEG-12 dimethicone, which is a copolymer of polydimethylsiloxane and polyoxyalkylene ether, as the conditioning agent.

There is a formulation for hair straightening [RU2623033C1 published on June 21, 2017 by UNICOSMETIC Co. Ltd.], containing PEG-12 dimethicone, a mixture of cyclopentasiloxane with dimethiconol in a ratio of 3:1, PEG/PPG - 15/15 dimethicone, dimethicone PEG-8 meadowfoamate, dimethicone emulsion with laureth-4, laureth-23 and salicylic acid as the conditioning agent.

However, frequent use of conditioners leads to undesirable results: they can not be completely removed from the hair, cause unpleasant tactile sensations, the hair starts to clot, there is a feeling of hair dirtiness and reduced volume. Traditional air conditioners solve only the external problem of dyed hair - loss of gloss and smoothness, but can not restore the hair strength.

The publication RU2452466C2 [published on June 10, 2012 by UNILEVER N.V.] mentions hair treatment formulations that include polymeric molecules having affinity for hair. The affinity is provided by at least one side chain terminating in a functional group that directionally binds with the hair fiber. This functional group is a non-cationic particle capable of specific interaction with the protein surface of non-covalent hair fiber having a bond energy in the range of 0.5 to 3 kcal/mol. The polymer is a partial ester of poly (hydroxyethyl acrylate) or poly (hydroxyethyl methacrylate) with 3,4-dihydroxybenzoic acid.

Examples of applicable non-covalent interactions include van der Waals interactions and hydrogen bonds.

Functional groups that directionally bond with the hair fiber include free amino groups and free phenolic hydroxyl groups.

The initial compounds which can subsequently be attached to the polymer main chain to introduce free amino groups include alkylamines (e.g., 1,3-diaminopropane, 1,6-hexanediamine, ethylenediamine, diethylenetriamine), unsaturated hydrocarbon amines (e.g., allylamine), aminoalcohols (e.g., ethanolamine, hydroxyamine), amidines (e.g., melamine), imines (e.g., polyethyleneimine), amino acids (e.g., tryptophan), polyamines, polyamides, alkaloids and mixtures thereof. The preferred example is the tryptophan amino acid. It is able to provide exceptional strength of bonding to the protein surface of the hair fiber.

The disclosed formulations provide deposition and transport of beneficial agents to the hair in the most effective way. However, these molecules are very large, which makes it difficult for them to penetrate into the hair and creates steric hindrance when delivering hair-beneficial agents to the target. At the same time, the problems of hair thinning in case of frequent coloring and reduction of their mechanical strength, including tensile strength, are still not solved.

The closest solution to the proposed invention for dealing with a problem of restoring properties of dyed hair is a technical solution disclosed in the publication US7211243B2 [published on May 01, 2007 by PROCTER & GAMBLE] and related to use of polysiloxane compositions with terminal amino functional groups for hair conditioning and their use in hair dye formulations. It is disclosed that polysiloxanes with molecules where amino groups are located in terminal positions of chains, have a much greater conditioning efficiency. It was shown that use of such polymers with terminal amino functional groups significantly reduces the stickiness and greasiness of the hair after treatment, while exhibiting excellent conditioning properties such as easy untangling, giving the hair glossy and healthy appearance. It is proposed to use polysiloxanes with terminal amino functional groups of the following general formula $R_{3-y}Q_ySiO[A]_xSiQ_zR_{3-z}$, where A represents $R_2SiO$,

R represents $C_1$-$C_5$ alkyl group, or phenyl group, or alkoxy group, or hydroxy group;

Q represents aminofunctional group of formula $-R^2Z$,

$R^2$ represents divalent $C_3$-$C_6$ alkylene radical,

Z represents $-N(R^3)_2$ or $-NR^3(CH_2)_nN(R^3)_2$,

$R^3$ represents independently H atom, or $C_1$-$C_{20}$ alkyl group, or phenyl or benzyl;

x is located between 70 and 150,

y is located between 1 and 3,

z is located between 1 and 3,

n is located between 2 and 6.

The results of sensory tests of compositions containing polysiloxanes with terminal amino functional groups demonstrate significant improvements in the conditioning profile, absence of sticking sensation or sensation of residue, and also easy untangling is ensured.

However, the polymers integrated in these compositions are very large and can only work on the hair surface, generally creating a visual effect and not solving the problem of hair strength loss during treatment, for example during and after coloring. Even in case of using these conditioners and an apparently hair appearance, the hair remains damaged in the inner layers, split and even break.

The problem of improving the hair mechanical strength is solved in the present invention.

SUBSTANCE OF THE INVENTION

The present invention solves the problem of providing new hair care formulations containing diamines of the homologous series of ethylenediamine.

In the first aspect, the present invention relates to a hair care product containing at least one $\alpha,\omega$-diamine of the general formula $H_2N-CH_2-(CH_2)_n-CH_2-NH_2$ or its salt, where n = 0, 1, 2, 3, 4 or 5, which improves the hair mechanical strength.

In an embodiment of the present invention, at least one specified $\alpha,\omega$-diamine is 1,2-ethylenediamine, 1,3-propylenediamine, 1,4-butanediamine, 1,5-pentanediamine, 1,6-hexanediamine or 1,7-heptanediamine or at least one of specified $\alpha,\omega$-diamine is 1,2-ethylenediamine, 1,3-propylenediamine or 1,6-hexanediamine.

In an embodiment of the present invention, at least one salt of at least one specified $\alpha,\omega$-diamine is a salt of sulfuric, hydrochloric, phosphoric, lactic, citric or acetic acids.

In an embodiment of the present invention, the specified product is a single-phase or multi-phase coloring formulation, perming formulation, straightening formulation, essence, balm, shampoo, conditioner, rinse, mask, tonic, lotion, milk, emulsion, gel, oil, gloss, wax, clay, paste, serum, cream, spray, mousse, foam or fluid.

In an embodiment of the present invention, the specified product is a multi-phase coloring formulation consisting of Phase A, Phase B and Phase C separated from each other and mixed immediately before use, where:

- Phase A contains alkaline agents, thickeners in the form of associated polymers, non-associated cross-linked polymers, polysaccharides, polycarboxylates, polyacrylates, or derivatives, or mixtures thereof; waxes, essential and mineral oils; cationic, anionic, non-ionic surfactants; cationic and anionic polymers; reducing agents in the form of inorganic salts; emulsifiers in the form of fatty alcohols, fatty esters, fatty acids; organic solvents in the form of mono- and polyatomic alcohols; silicones, preservative agents, moisturizing agents, emollients, complexones, fragrances, oxidation coloring components, color components such as m-aminophenols, di- and trihydroxy derivatives of the aromatic and heterocyclic series, nitro-, chloro-, methoxy- and ethoxy derivatives thereof; direct dyes, such as acidic, basic, cationic, active, dispersed, vat dyes, which are nitric, anthraquinone, azo, arylmethane, arylamine, phthalocyanine, indigoid, and thioindigoid dyes, and their heterocyclic analogues; water;

- Phase B contains hydrogen peroxide, thickeners, emulsifiers, emollients, surfactants, complexones;
- Phase C contains at least one specified $\alpha,\omega$-diamine, cationic surfactants, moisturizing agents, silicones, thickeners, preservative agents, water.

In an embodiment of the present invention, at least one specified $\alpha,\omega$-diamine or its salt is present in an amount of about 1-50% by weight, preferentially about 3-40% by weight, more preferentially about 5-30% by weight, most preferentially about 8-20% by weight.

In an embodiment of the present invention, the hair care product may additionally contain cetrimonium chloride, panthenol, quaternium-18 silicone emulsion with trideceth-6 and trideceth-12, hydroxyethylcellulose, preservative agent, water.

In an embodiment of the present invention, the hair care product may contain, % by weight:

| | |
|---|---|
| specified $\alpha,\omega$-diamine | from about 8.0 to about 20.0; |
| cetrimonium chloride | from about 0.5 to about 10.0; |
| panthenol | from about 1.5 to about 10.0; |
| quaternium-18 silicone emulsion with trideceth-6 and trideceth-12 | from about 1.5 to about 10.0; |
| hydroxyethylcellulose | from about 0.05 to about 1.0; |
| preservative agent | from about 0.01 to about 1.0; |
| water | up to 100. |

In the second aspect, the present invention relates to use of at least one $\alpha,\omega$-diamine of the general formula $H_2N-CH_2-(CH_2)_n-CH_2-NH_2$ or its salt, where n = 0, 1, 2, 3, 4 or 5, in the hair care product to improve the hair mechanical strength.

In an embodiment of the present invention, at least one specified $\alpha,\omega$-diamine is 1,2-ethylenediamine, 1,3-propylenediamine, 1,4-butanediamine, 1,5-pentanediamine, 1,6-hexanediamine or 1,7-heptanediamine or at least one of specified $\alpha,\omega$-diamine is 1,2-ethylenediamine, 1,3-propylenediamine or 1,6-hexanediamine.

In an embodiment of the present invention, at least one salt of at least one specified $\alpha,\omega$-diamine is a salt of sulfuric, hydrochloric, phosphoric, lactic, citric or acetic acids.

In the third aspect, the present invention relates to use of at least one $\alpha,\omega$-diamine of the general formula $H_2N-CH_2-(CH_2)_n-CH_2-NH_2$ or its salt, where n = 0, 1, 2, 3, 4 or 5, to improve the hair mechanical strength.

In an embodiment of the present invention, at least one specified $\alpha,\omega$-diamine is 1,2-ethylenediamine, 1,3-propylenediamine, 1,4-butanediamine, 1,5-pentanediamine, 1,6-hexanediamine or 1,7-heptanediamine or at least one of specified $\alpha,\omega$-diamine is 1,2-ethylenediamine, 1,3-propylenediamine or 1,6-hexanediamine.

In an embodiment of the present invention, at least one salt of at least one specified $\alpha,\omega$-diamine is a salt of sulfuric, hydrochloric, phosphoric, lactic, citric or acetic acids.

The technical result of the present invention is the increase of the hair mechanical strength by using $\alpha,\omega$-diamines of the invention either alone or in combination with other additive agents.

Another technical result of the present invention is the provision of hair care products that improve the hair mechanical strength by incorporating $\alpha,\omega$-diamines in the hair.

Another technical result of the present invention is the provision of hair care products that improve the hair mechanical strength by incorporating in the hair $\alpha,\omega$-diamines that improve untangling and intensify the hair gloss due to introduction of cetrimonium chloride and quaternium-18 silicone emulsion with trideceth-6 and trideceth-12, and enhance the hair moisture due to incorporation of panthenol into the hair.

Unless otherwise specified, all technical and scientific terms used herein have the meaning understandable by a person with average skills in the art of invention.

Other characteristics and advantages of the present invention will be clarified in the following detailed description and formula of the invention.

## DETAILED DESCRIPTION OF THE INVENTION

Terms and definitions used.

- $\alpha,\omega$-diamine $H_2N-CH_2-(CH_2)_n-CH_2-NH_2$ is a 1,2-ethylenediamine (n = 0), 1,3-propylenediamine (n = 1), 1,4-butanediamine (n = 2), 1,5-pentanediamine (n = 3), 1,6-hexanediamine (n = 4) or 1,7-heptanediamine (n = 5).
- 1,2-ethylenediamine: $H_2NCH_2CH_2NH_2$: synonyms ethylenediamine, 1,2-diaminoethane, ethanediamine, ethane-1,2-diamine.
- 1,3-propylenediamine $H_2NCH_2CH_2CH_2NH_2$: synonyms propylenediamine, trimethylenediamine, 1,3-diaminopropane, propanediamine, propane-1,3-diamine.
- 1,4-butanediamine $H_2NCH_2CH_2CH_2CH_2NH_2$: synonyms butylenediamine, tetramethylenediamine, 1,4-diaminobutane, butane-1,4-diamine.
- 1,5-pentanediamine $H_2NCH_2CH_2CH_2CH_2CH_2NH_2$: synonyms pentamethylenediamine, 1,5-diaminopentane, pentane-1,5-diamine.
- 1,6-hexanediamine $H_2NCH_2CH_2CH_2CH_2CH_2CH_2NH_2$: synonyms hexamethylenediamine, 1,6-diaminohexane, hexane-1,6-diamine.
- 1,7-heptanediamine $H_2NCH_2CH_2CH_2CH_2CH_2CH_2CH_2NH_2$: synonyms heptamethylenediamine, 1,7-diaminoheptane, heptane-1,7-diamine.

At least one salt of at least one specified $\alpha,\omega$-diamine is a salt of sulfuric, hydrochloric, phosphoric, lactic, citric, acetic or any other cosmetically acceptable acid. Salts of $\alpha,\omega$-diamines of the general formula $H_2N-CH_2-(CH_2)_n-CH_2-NH_2$, where n = 0, 1, 2, 3, 4 or 5, are derivatives containing a diamine cation and an anionic acid residue.

Single-phase or multi-phase coloring formulation refers to a hair care product designed to change the hair natural color.

Chemical waving formulation refers to a product for creating waves or curls.

Straightening formulation refers to a product for curly hair straightening.

Essence refers to a concentrated hair care product in the form of an emulsion cream or liquid gel.

Balm refers to a product that has a beneficial (moisturizing, soothing, toning, etc.) effect.

Shampoo refers to a product intended for hair and scalp washing.

Conditioner refers to a hair care product used after washing to improve the hair combing and/or to give it volume and gloss.

Mask refers to a product that has a beneficial (moisturizing, soothing, toning, etc.) effect.

Tonic refers to a scalp and hair care product in the form of an aqueous, aqueous-alcoholic, alcoholic solution.

Lotion refers to a scalp and hair care product in the form of an aqueous, aqueous-alcoholic, alcoholic solution.

Milk refers to a care product in the form of a liquid emulsion cream.

Emulsion refers to a perfumery and cosmetic product, which is a colloidal system.

Gel refers to a perfumery and cosmetic product with gel consistency intended for hair care and coloring.

Oil refers to a perfumery and cosmetic product based on vegetable and/or mineral oils.

Gloss relates to perfumery and cosmetic product, including decorative cosmetics product, intended to improve the hair gloss.

Wax refers to a perfumery and cosmetic product manufactured using film-forming substances and natural or synthetic waxes.

Clay refers to a perfumery and cosmetic product based on natural mineral substances.

Paste refers to a pasteous perfumery and cosmetic product.

Serum refers to a perfumery and cosmetic product containing a complex of active substances that provides a cosmetic effect.

Cream refers to a creamy perfumery and cosmetic product.

Spray refers to a perfume and cosmetics hair care product intended to hold the hair when styling and to give the hair gloss and aroma.

Mousse refers to a perfumery and cosmetic product containing a large number of air bubbles.

Foam refers to a foamy perfumery and cosmetic product or a product that forms foam during application.

Fluid refers to a care product in the form of a liquid emulsion cream or gel.

Mechanical strength characterizes the material property to resist breakdown under mechanical stress. It is characterized, in the case of elastic mono fibers, which include hair, by yield strength, breaking strength and breaking elongation. Yield strength is a mechanical characteristic of a material characterizing the stress at which deformations continue to grow without load increase. Breaking strength is a characteristic of a material characterizing the stress at which the material breaks. Breaking elongation shows how much the initial fiber length changes at the moment of reaching the breaking strength. It characterizes the elastic deformation.

At least one α,ω-diamine of the invention or its salt may be present in the hair care product in an amount of about 1-50% by weight, preferentially about 3-40% by weight, more preferentially about 5-30% by weight, most preferentially about 8-20% by weight. Therefore, at least one α,ω-diamine of the invention or its salt may be contained in the hair care product in an amount of about 1, 5, 10, 15, 20, 25, 30, 35, 40, 45 or 50% by weight, or in an amount of about 1-5, 5-10, 10-15, 15-20, 20-25, 25-30, 30-35, 35-40, 40-45 or 45-50% by weight. Alternatively, at least one α,ω-diamine of the invention or its salt may be contained in the hair care product in an amount of 1, 5, 10, 15, 20, 25, 30, 35, 40, 45 or 50% by weight, or in an amount of about 1-5, 5-10, 10-15, 15-20, 20-25, 25-30, 30-35, 35-40, 40-45 or 45-50% by weight.

Additionally, the hair care product providing hair strength may contain, % by weight:

| | |
|---|---|
| α,ω-diamine of the invention | from about 8.0 to about 20.0; |
| cetrimonium chloride | from about 0.5 to about 10.0; |
| panthenol | from about 1.5 to about 10.0; |
| quaternium-18 silicone emulsion with trideceth-6 and trideceth-12 | from about 1.5 to about 10.0; |
| hydroxyethylcellulose | from about 0.05 to about 1.0; |
| preservative agent | from about 0.01 to about 1.0; |
| water | up to 100. |

Therefore, the hair care product providing hair strength may contain the α,ω-diamine of the invention in an amount of about 8.0-20.0% by weight, 10.0-18.0% by weight, 12.0-16.0% by weight, 14.0% by weight. Alternatively, the hair care product providing hair strength may contain the α,ω-diamine of the invention in an amount of about 8, 9, 10, 11, 12, 13, 14, 15, 16, 17, 18, 19 or 20% by weight.

Therefore, the hair care product providing hair strength may contain cetrimonium chloride in an amount of about 0.5-10.0% by weight, 1.0-9.0% by weight, 3.0-7.0% by weight, 5.0% by weight. Alternatively, the hair care product providing hair strength may contain cetrimonium chloride in an amount of about 0.5, 0.6, 0.7, 0.8, 0.9, 1, 2, 3, 4, 5, 6, 7, 8, 9 or 10% by weight.

Therefore, the hair care product providing hair strength may contain panthenol in an amount of about 1.5-10.0% by weight, 3.0-7.0% by weight, 5.0% by weight. Alternatively, the hair care product providing hair strength may contain panthenol in an amount of about 1.5, 2, 3, 4, 5, 6, 7, 8, 9 or 10% by weight.

Therefore, the hair care product providing hair strength may contain quaternium-18 silicone emulsion with trideceth-6 and trideceth-12 in an amount of about 1.5-10.0% by weight, 3.0-7.0% by weight, 5.0% by weight. Alternatively, the hair care product providing hair strength may contain panthenol in an amount of about 1.5, 2, 3, 4, 5, 6, 7, 8, 9 or 10% by weight.

Therefore, the hair care product providing hair strength may contain hydroxyethylcellulose in an amount of about 0.05-1.0% by weight, 0.1-1.0% by weight, 0.3-0.7% by weight, 0.4% by weight. Alternatively, the hair care product providing hair strength may contain hydroxyethylcellulose in an amount of about 0.05, 0.06, 0.07, 0.08, 0.09, 0.1, 0.2, 0.3, 0.4, 0.5, 0.6, 0.7, 0.8, 0.9 or 1% by weight.

Therefore, the hair care product providing hair strength may contain a preservative agent in an amount of about 0.01-1.0% by weight, 0.03-1.0% by weight, 0.05-1.0% by weight, 0.1-1.0% by weight, 0.3-0.7% by weight, 0.4% by weight. Alternatively, the hair care product providing hair strength may contain a preservative agent in an amount of about 0.01, 0.02, 0.03, 0.04, 0.05, 0.06, 0.07, 0.08, 0.09, 0.1, 0.2, 0.3, 0.4, 0.5, 0.6, 0.7, 0.8, 0.9 or 1% by weight.

Or the hair care product providing hair strength may contain:

| | |
|---|---|
| specified α,ω-diamine | 8.0-20.0; |
| cetrimonium chloride | 0.5-10.0; |
| panthenol | 1.5-10.0; |
| quaternium-18 silicone emulsion with trideceth-6 and trideceth-12 | 1.5-10.0; |
| hydroxyethylcellulose | 0.05-1.0; |
| preservative agent | 0.01-1.0; |
| water | up to 100 |

At least one α,ω-diamine of the invention or its salt may be used in the hair care product, included in the hair care product, used to prepare the hair care product, or at least one α,ω-diamine of the invention or its salt may be used to improve the hair mechanical strength.

Examples

The experiments for studying stress-related characteristics of examined materials in the mode of uniaxial tension were conducted on an Instron-1122 universal machine. This device makes it possible to vary loading rate from 0.05 mm/min to 1000 mm/min and the load range from $10^{-3}$N to $5\ 10^{3}$N. The recording instrument of the machine records the diagram of tension as a function of elongation $P(\Delta l)$ (where P - load, N; $\Delta l$ - absolute elongation of a sample, mm). The samples were tested at the specified elongation rate of 10 mm/min. The obtained data were transformed into functions $\sigma(\varepsilon)$ ($\sigma$ - tension stress, N/mm$^2$, $\varepsilon$ - relative elongation, %), where:

$$\sigma_i = F/P,$$

where F - cross-section area of human hair that is determined as:

$$F = \pi \cdot D_i^2 / 4, \ mm^2,$$

where $D_i$ - diameter of hair, mm.

$$\varepsilon_i = \Delta 1 / l_0 \cdot 100\%,$$

where $l_0$ - initial length of a sample, equal to 30 mm.
An averaged load-elongation curve for a single human hair was obtained with the help of mathematical statistics methods.
On the basis of obtained load-elongation curves basic mechanical characteristics were determined: yield strength ($\sigma_\tau$, MPa), breaking strength ($\sigma_\pi$, MPa), breaking elongation ($\varepsilon_p$, %) and initial tangent modulus of elasticity ($E_o$, GPa).
The values of initial tangent modulus of elasticity $E_0$ were determined by elongation curve derivation, i.e.:

$$E_{0=} \partial\sigma_\tau(\varepsilon_p)/\partial\varepsilon_p.$$

During the tests, the formulations listed in Table 1 were used. Also, positive results for 1,4-butanediamine, 1,5-pentanediamine and 1,7-heptanediamine were obtained, but these chemical compounds have a characteristic smell, so their use in cosmetics may be difficult.

Table 1. Formulations.

| Component name | Content of components, % by weight | | | | | |
|---|---|---|---|---|---|---|
| | 1 | 2 | 3 | 4 | 5 | 6 |
| 1,6-hexanediamine | 8.0 | 20.0 | - | - | - | - |
| 1,2-ethylenediamine | - | - | 8.0 | 20.0 | - | - |
| 1,3 -prop ylenediamine | - | - | - | - | 8.0 | 20.0 |
| cetrimonium chloride | 0.8 | 0.8 | 2.5 | 2.5 | 1.5 | 1.5 |
| panthenol | 2.0 | 2.0 | 5.0 | 5.0 | 8.0 | 8.0 |
| quaternium-18 silicone emulsion with trideceth-6 and trideceth-12 | 3.0 | 3.0 | 6.0 | 6.0 | 8.0 | 8.0 |
| hydroxyethylcellulose | 0.08 | 0.08 | 0.3 | 0.3 | 0.6 | 0.6 |
| preservative agent | 0.05 | 0.05 | 0.5 | 0.5 | 0.8 | 0.8 |
| water | up to 100 | up to 100 | up to 100 | up to 100 | up to 100 | up to 100 |

The hair treatment was performed using a coloring formulation and composition. 60 g of hair color were mixed with 60 g of revelator, than 5 g of a composition from Table 1 were added, the mixture was stirred until achieving complete homogeneity and applied to the hair. After leaving for 40 minutes, the composition was washed off with shampoo and the hair was blow dried.

The tests of human hair colored with and without introduction of the Table 1 composition of the invention into the color formulation showed that in the former case a significant increase in the hair strength (mechanical) characteristics occurs.

Table 2 shows the results of uncolored hair tests, calculated with a confidence coefficient of 95%.

Table 2. Deformation-strength properties of uncolored human hair samples.

| Sample No. | Hair average diameter, mm | Yield strength, MPa | Breaking strength, MPa | Breaking elongation, % | Initial tangent modulus of elasticity, GPa |
|---|---|---|---|---|---|
| 1ref | 0.053 | 256 ± 14 | 419 ± 25 | 47 ± 2 | 9.8 ± 0.6 |
| 2ref | 0.044 | 246 ± 30 | 397 ± 48 | 48 ± 2 | 9.3 ± 1 |
| 3ref | 0.047 | 268 ± 23 | 450 ± 40 | 48 ± 2 | 11 ± 1 |
| 4ref | 0.069 | 212 ± 8 | 344 ± 9 | 54 ± 3 | 8 ± 0.6 |
| 5ref | 0.059 | 230 ± 31 | 363 ± 46 | 48 ± 1 | 9 ± 1 |
| 6ref | 0.063 | 270 ± 16 | 404 ± 49 | 47 ± 8 | 11.6 ± 1.7 |
| 7ref | 0.053 | 198 ± 11 | 329 ± 18 | 45 ± 3 | 8.4 ± 1.1 |
| 8ref | 0.063 | 190 ± 14 | 344 ± 28 | 50 ± 3 | 7.7 ± 0.7 |
| 9ref | 0.063 | 243 ± 27 | 423 ± 126 | 49 ± 3 | 9.0 ± 2.6 |
| 10ref | 0.047 | 194 ± 28 | 338 ± 36 | 50 ± 6 | 8.3 ± 1.0 |

Based on the data obtained for further treatment with coloring formulations, hair samples No. 3ref, 5ref, 6ref and 9ref, which demonstrated the highest deformation-strength characteristics, were selected.

Table 3 shows the data on mechanical testing of hair treated with coloring formulations without the composition of the invention (samples $3^0$, $5^0$, $6^0$ and $9^0$), coloring formulations with addition of the composition of the invention containing 1,2-ethylenediamine in a concentration of 10% (samples $3^1$, $5^1$) and 20% (samples $3^2$, $5^2$), calculated with a confidence coefficient of 95%.

Table 3. Deformation-strength properties of human hair samples after treatment with a formulation containing 1,2-ethylenediamine.

| Sample No. | Average diameter, mm | Yield strength, MPa | Breaking strength, MPa | Breaking elongation, % | Initial tangent modulus of elasticity, GPa |
|---|---|---|---|---|---|
| $3^0$ | 0.057 | 248 ± 23 | 419 ± 36 | 49 ± 3 | 8.0 ± 0.8 |
| $3^1$ | 0.056 | 247 ± 23 | 420 ± 38 | 48 ± 3 | 9.0 ± 1.0 |
| $3^2$ | 0.0565 | 253 ± 21 | 404 ± 37 | 46 ± 3 | 9.8 ± 0.8 |
| $5^0$ | 0.061 | 194 ± 17 | 329 ± 31 | 46 ± 4 | 6.6 ± 0.6 |
| 51 | 0.062 | 224 ± 20 | 365 ± 32 | 45 ± 3 | 9.6 ± 1.0 |
| $5^2$ | 0.058 | 231 ± 22 | 374 ± 36 | 45 ± 4 | 9.9 ± 1.0 |

Table 4 shows the data on mechanical testing of hair treated with coloring formulations without the composition of the invention (samples $3^0$, $5^0$, $6^0$ and $9^0$), coloring formulations with addition of the composition of the invention containing 1,6-hexanediamine in a concentration of 10% (samples $6^1$, $9^1$) and 20% (samples $6^2$, $9^2$), calculated with a confidence coefficient of 95%.

Table 4. Deformation-strength properties of human hair samples after treatment with a formulation containing 1,6-hexanediamine.

| Sample No. | Average diameter, mm | Yield strength, MPa | Breaking strength, MPa | Breaking elongation, % | Initial tangent modulus of elasticity, GPa |
|---|---|---|---|---|---|
| $6^0$ | 0.056 | $204 \pm 19$ | $357 \pm 34$ | $49 \pm 4$ | $6.8 \pm 0.8$ |
| $6^1$ | 0.054 | $223 \pm 21$ | $371 \pm 40$ | $48 \pm 3$ | $9.2 \pm 0.9$ |
| $6^2$ | 0.054 | $230 \pm 25$ | $389 \pm 50$ | $49 \pm 4$ | $9.5 \pm 1.1$ |
| $9^0$ | 0.057 | $245 \pm 22$ | $424 \pm 31$ | $51 \pm 4$ | $8 \pm 0.8$ |
| $9^1$ | 0.057 | $280 \pm 30$ | $453 \pm 58$ | $45 \pm 4$ | $11.4 \pm 1.0$ |
| $9^2$ | 0.058 | $295 \pm 25$ | $455 \pm 54$ | $47 \pm 2$ | $11.7 \pm 1.0$ |

As it seen from the obtained data, use of coloring formulations without the composition of the invention results in a decrease in the yield strength, breaking strength and initial tangent modulus of elasticity on average from 15 to 30%. The breaking elongation remained at the same level of about 50%. In case of coloring the samples with coloring formulations containing the composition of the invention, with increase of their concentration, an improvement in the yield strength, breaking strength and tangent modulus of elasticity in comparison with human hair colored with commercial hair color is observed; breaking elongation is slightly reduced.

Thus, it may be unambiguously concluded that introduction of $\alpha,\omega$-diamines of the invention into the coloring formulation improves the key parameters of the hair mechanical strength.

Also, studies of the effect on the hair of $\alpha,\omega$-diamines of the invention in the form of aqueous diamine solutions were performed. Hair treated with a coloring formulation containing aqueous diamine solutions was examined to exclude the influence of the remaining components (conditioning agents) of the invention compositions. The findings are presented in Tables 5 and 6.

Table 5 shows the data on mechanical testing of hair treated with coloring formulations without the composition of the invention (samples $3^0$, $5^0$), coloring formulations with addition of an aqueous solution of 1,2-ethylenediamine in a concentration of 10% (samples $3^{10\%}$, $5^{10\%}$) and 20% (samples $3^{20\%}$, $5^{20\%}$), calculated with a confidence coefficient of 95%.

Table 5. Deformation-strength properties of human hair samples after treatment with an aqueous solution of 1,2-ethylenediamine.

| Sample No. | Average diameter, mm | Yield strength, MPa | Breaking strength, MPa | Breaking elongation, % | Initial tangent modulus of elasticity, GPa |
|---|---|---|---|---|---|
| $3^0$ | 0.057 | $248 \pm 23$ | $419 \pm 36$ | $49 \pm 3$ | $8.0 \pm 0.8$ |
| $3^{10\%}$ | 0.058 | $266 \pm 13$ | $452 \pm 20$ | $49 \pm 3$ | $11 \pm 0.7$ |
| $3^{20\%}$ | 0.061 | $255 \pm 20$ | $435 \pm 35$ | $48 \pm 3$ | $11 \pm 0.8$ |
| $5^0$ | 0.061 | $194 \pm 17$ | $329 \pm 31$ | $46 \pm 4$ | $6.6 \pm 0.6$ |
| $5^{10\%}$ | 0.0555 | $202 \pm 13$ | $346 \pm 17$ | $47 \pm 3$ | $8.6 \pm 0.7$ |
| $5^{20\%}$ | 0.052 | $247 \pm 35$ | $434 \pm 61$ | $48 \pm 3$ | $10 \pm 1.3$ |

Table 6 shows the data on mechanical testing of hair treated with coloring formulations without the composition of the invention (samples $6^0$, $9^0$), coloring formulations with addition of an aqueous solution of 1,2-hexanediamine in a concentration of 10% (samples $6^{10\%}$, $9^{10\%}$) and 20% (samples $6^{20\%}$, $9^{20\%}$), calculated with a confidence coefficient of 95%.

Table 6. Deformation-strength properties of human hair samples after treatment with an aqueous solution of 1,6-hexanediamine.

| Sample No. | Average diameter, mm | Yield strength, MPa | Breaking strength, MPa | Breaking elongation, % | Initial tangent modulus of elasticity, GPa |
|---|---|---|---|---|---|
| $6^0$ | 0.056 | 204 ± 19 | 357 ± 34 | 49 ± 4 | 6.8 ± 0.8 |
| $6^{10\%}$ | 0.063 | 235 ± 25 | 338 ± 19 | 41 ± 2 | 9 ± 0.6 |
| $6^{20\%}$ | 0.060 | 206 ± 15 | 344 ± 38 | 43 ± 3 | 9.6 ± 0.4 |
| $9^0$ | 0.057 | 245 ± 22 | 424 ± 31 | 51 ± 4 | 8 ± 0.8 |
| $9^{10\%}$ | 0.058 | 249 ± 22 | 459 ± 28 | 51 ± 2 | 10 ± 0.9 |
| $9^{20\%}$ | 0.062 | 267 ± 31 | 480 ± 73 | 51 ± 3 | 10.4 ± 0.7 |

In order to exclude the influence of the coloring formulation components, studies of hair treated with only the composition of the invention were performed.

The obtained data were compared with the results of tests of hair treated with the coloring formulation containing the composition of the invention (see Tables 3 and 4). The results are calculated with a confidence coefficient of 95% and presented in Tables 7 and 8.

Table 7. Study of the effect of a composition containing 1,2-ethylenediamine.

| Sample No. | Average diameter, mm | Yield strength, MPa | Breaking strength, MPa | Breaking elongation, % | Initial tangent modulus of elasticity, GPa |
|---|---|---|---|---|---|
| $5^{I}$ | 0.050 | 217 ± 29 | 405 ± 60 | 52 ± 2 | 9.9 ± 1.4 |
| $5^{II}$ | 0.062 | 224 ± 20 | 365 ± 32 | 45 ± 3 | 9.6 ± 1.0 |
| $5^{I}$ - sample treated with a composition containing 10% 1,2-ethylenediamine. $5^{II}$ - sample treated with a coloring formulation containing a composition with 10% ethylenediamine. | | | | | |

Table 8. Study of the effect of a composition containing 1,6-hexanediamine.

| Sample No. | Average diameter, mm | Yield strength, MPa | Breaking strength, MPa | Breaking elongation, % | Initial tangent modulus of elasticity, GPa |
|---|---|---|---|---|---|
| $6^{I}$ | 0.066 | 238 ± 14 | 329 ± 16 | 40 ± 3 | 9.1 ± 0.4 |
| $6^{II}$ | 0.063 | 235 ± 25 | 338 ± 19 | 41 ± 2 | 9 ± 0.6 |
| $6^{I}$ - sample treated with a composition containing 10% 1,6-hexanediamine. $6^{II}$ - sample treated with a coloring formulation containing a composition with 10% 1,6-hexanediamine. | | | | | |

Thus, it may be unambiguously concluded that the composition of the invention improves the hair yield strength, breaking strength, breaking elongation and initial tangent modulus of elasticity.

Preparation of hair coloring formulation.

Creamy Emulsion (Phase A)

*Preparation of fat phase melt.*

Fat components: waxes, essential and mineral oils, emulsifiers in the form of fatty alcohols, fatty esters, fatty acids, cationic, anionic, nonionic surfactants are melted at 75-80 °C and stirred for 30 minutes at this temperature.

*Preparation of aqueous solution of dyes.*

The calculated amount of water is heated to 45-50 °C, salts of reducing agents, complexone, organic solvents in the form of mono- and polyatomic alcohols, cationic and anionic polymers, dyes are added, argon is supplied, the mixture is heated to 75 °C and left for 60 minutes.

The fat phase is mixed with the aqueous solution of dyes at a temperature of 65-75 °C and left for 30 minutes, then the mixture is cooled to 40 °C and thermolabile components, such as silicones, fragrance composition, preservative agent, ammonium hydroxide and others are added. The formulation is stirred for about 30 minutes, then a sample is taken for analysis. In the event of a positive result, the finished product is packed in tubes.

Oxidizing Agent (Phase B)

The calculated amount of water is heated to 45-50 °C, thickeners, emulsifiers, emollients, surfactants are added and the mass is stirred for 60 minutes. Then the mass is cooled to 30 °C and complexone, acids, hydrogen peroxide are added. The mass is stirred for about 30 minutes, then a sample is taken for analysis. In the event of a positive result, the finished product is packed in bottles.

Composition containing diamine and other additives (Phase C)

The calculated amount of water is heated to 45-50 °C, hydroxyethylcellulose is added and the mixture is homogenized. Then it is cooled to 25-30 °C and diamine, silicone emulsion, cetrimonium chloride, panthenol, preservative agent are added. The mass is stirred for 30 minutes, a sample is taken for analysis, and in the event of positive result the product is packed in special bottles.

Phase A is mixed with Phase B and Phase C immediately prior to use.

Preparation of hair shampoo.

Anionic, cationic, non-ionic surfactants, moisturizing and conditioning additives and diamine are added to the calculated amount of water. The mixture is heated to 35-40 °C and mixed for 60-90 minutes to achieve complete homogenization. Then it is cooled to 30 °C and preservative agent, perfume, pH modifiers, complexone, thickeners and solubilizers are added. The mixture is stirred for 60 minutes to achieve complete homogenization. Then a sample is taken for analysis, and in the event of positive result the product is packed in special bottles.

Preparation of chemical hair perming product.

The calculated amount of water is loaded with pH modifier and thoroughly stirred. Then perfume, solubilizer, moisturizing and conditioning additives, anionic, cationic, non-ionic surfactants, preservative agent are added. The mixture is stirred for 30 minutes until complete dissolution. Diamine, ammonium thioglycollate are added and the mixture is stirred for 60 minutes until complete dissolution. The calculated amount of ammonia is added, mixture is stirred for 15 minutes. Then a sample is taken for analysis, and in the event of positive result the product is packed in special bottles.

At least one $\alpha,\omega$-diamine of the general formula $H_2N\text{-}CH_2\text{-}(CH_2)_n\text{-}CH_2\text{-}NH_2$ or its salt, where $n = 0, 1, 2, 3, 4$ or 5, in an amount of about 1-50% by weight, preferentially about 3-40% by weight, more preferentially about 5-30% by weight, most preferentially about 8-20% by weight may be included, for example, in a hair straightening formulation, which is an emulsion that is activated at a temperature of 210-230 °C and contains straightening agent glyoxylic acid, emulsifier, conditioning agent, perfume composition, preservative and water, characterized by the fact that, in order to obtain a formulation that provides the effect of straightened hair throughout a year while maintaining hair high quality, has a neutral odor and is easily removable from the hair and does not prevent intensive hair coloring with oxidation dyes immediately after straightening, non-ionic surfactants are used as emulsifiers, and non-ionic silicones are used as the conditioning agent, in % by weight:

| | |
|---|---|
| straightening agent: glyoxylic acid | 12.0-20.0; |
| nonionic surfactant emulsifier: | |
| ceteareth-6, mixture of ceteareth-20 or ceteareth-25 with cetostearyl alcohol in a ratio of 10:1 and others | 4.0-8.0; |
| non-ionic silicone conditioning agent: | |
| PEG-12 dimethicone, mixture of cyclopentasiloxane with dimethiconol in a ratio of 3:1, PEG/PPG-15/15 dimethicone, dimethicone PEG-8 meadowfoamate, dimethicone emulsion with laureth-4, laureth-23 and salicylic acid | 3.0-7.0; |
| perfume composition | 0.1-1.0; |
| preservative agent | 0.01-0.5; |
| water | up to 100. |

At least one α,ω-diamine of the general formula $H_2N-CH_2-(CH_2)_n-CH_2-NH_2$ or its salt, where n = 0, 1, 2, 3, 4 or 5, in an amount of about 1-50% by weight, preferentially about 3-40% by weight, more preferentially about 5-30% by weight, most preferentially about 8-20% by weight may be included, for example, in a foam hair coloring formulation, characterized by the fact that it consists of two phases - Phase A and Phase B, placed separately and mixed immediately prior to use, where Phase B is an oxidizing agent in the form of aqueous solution containing hydrogen peroxide, pH 3.5; and Phase A is an emulsion containing a composition of anionic, nonionic and amphoteric surfactants combined in a specific ratio, as well as oxidation dyes and/or their salts in aqueous alkaline medium and/or direct dyes, alkaline agents providing a pH of 9.2-10.8; the anionic surfactant is a mixture of sodium laureth sulfate and laureth-5 sodium carboxylate; the nonionic surfactant is a mixture of cocamide DEA and decylglucoside; the amphoteric surfactant is cocamidopropyl betaine; the conditioning agent is PEG-12 dimethicone; the reducing agents are sodium sulfite, sodium erythorbate, sodium hydrosulfite; the water-softening complexone is tetrasodium salt of ethylenediaminetetraacetic acid; perfume composition and water at the following proportions of components in the specified emulsion; in % by weight:

| | |
|---|---|
| anionic surfactants: | |
| mixture of sodium laureth sulfate and laureth-5 sodium carboxylate in a ratio of 2:1 | 2.0-14.0; |
| non-ionic surfactants: | |
| mixture of cocamide DEA and decylglucoside in a ratio of 1:1 | 0.2-8.0; |
| amphoteric surfactant: | |
| cocamidopropyl betaine | 1.0-6.0; |
| coloring component | 0.0001-10.0; |
| conditioning agent: | |
| PEG-12 dimethicone | 0.1-5.0; |
| reducing agent: | |
| sodium sulfite, sodium erythorbate, sodium hydrogen sulphite | 0.11-2.5; |
| perfume composition | 0.1-1.2; |
| complexone: | |
| tetrasodium salt of ethylenediaminetetraacetic acid | 0.01-1.0; |
| water | up to 100. |

At least one α,ω-diamine of the general formula $H_2N-CH_2-(CH_2)_n-CH_2-NH_2$ or its salt, where n = 0, 1, 2, 3, 4 or 5, in an amount of about 1-50% by weight, preferentially about 3-40% by weight, more preferentially about 5-30% by weight, most preferentially about 8-20% by weight may be included, for example, in a hair coloring formulation, characterized by the fact that it consists of 2 phases placed separately and mixed immediately prior to use, where one phase is an oxidizing agent in the form of aqueous solution containing hydrogen peroxide, pH 3.5; and other phase includes a mixture of aminomethylpropanol and ammonium sulfate forming a buffer at pH of 10-10.5, which is introduced into a creamy emulsion including cetearyl alcohol as a thickening agent, surfactants (glycerol monostearate, ceteareth-30, cocamide MEA or PEG -40 hydrogenated castor oil) as emulsifiers; oxidation dyes and/or their salts with inorganic acids and/or direct dyes as coloring components; hexildecanol, hexyldecyl laurate, propylene glycol or ethoxydiglycol as organic solvents; sodium erythorbate and sodium sulphite as reducing agents; tetrasodium salt of ethylenediaminetetraacetic acid as a water-softening complexone; quaternium-70, hydrolyzed keratin, avocado oil, or dimethicone as conditioning agents; methylparaben, ethylparaben or propylparaben in phenoxyethanol as preservative agents; panthenol or olive extract as vitamins and provitamins; mica or titanium dioxide as agents giving pearly shine, and perfume composition at the following proportions of components in the specified emulsion; in % by weight:

| | |
|---|---|
| thickening agent: cetearyl alcohol | 6.0-10.0; |
| emulsifiers: | |
| glycerol monostearate, ceteareth-30, cocamide MEA, PEG-40 hydrogenated castor oil | 7.5-25.0; |
| organic solvent: | |
| hexildecanol, hexyldecyl laurate, propylene glycol, ethoxydiglycol | 3.5-11.0; |
| conditioning agent: | |
| quaternium-70, hydrolyzed keratin, avocado oil, dimethicone | 2.3-12.0; |
| reducing agent: | |
| sodium sulfite, sodium erythorbate | 0.3-1.4; |
| perfume composition | 0.2-0.5; |
| preservative agent: | |

(continued)

| | |
|---|---|
| methylparaben, ethylparaben, propylparaben in phenoxyethanol | 0.1-0.6; |
| complexone: tetrasodium salt of ethylenediaminetetraacetic acid | 0.01-0.1; |
| provitamins and vitamins: panthenol, olive extract | 0.5-3.0; |
| mica, titanium dioxide | 0.5-0.8; |
| water | up to 100 |

At least one $\alpha,\omega$-diamine of the general formula $H_2N-CH_2-(CH_2)_n-CH_2-NH_2$ or its salt, where n = 0, 1, 2, 3, 4 or 5, in an amount of about 1-50% by weight, preferentially about 3-40% by weight, more preferentially about 5-30% by weight, most preferentially about 8-20% by weight may be included, for example, in a hair coloring formulation, characterized by the fact that it consists of 2 phases - Phase A and Phase B, placed separately and mixed immediately prior to use, where Phase B is an oxidizing agent in the form of aqueous solution containing hydrogen peroxide, pH 3.5; and Phase A containing cationic polymers and cationic surfactants, includes a mixture of alkaline ammonium hydroxide agents (25% solution), and a non-volatile base of aminomethylpropanol creating a pH of 9.2-10.8 during the entire coloring period (40-50 minutes), resulting in intense, deep and persistent colors, introduced into a creamy emulsion, which contains oxidation dyes and/or their salts with inorganic acids, and/or direct dyes as coloring components; carnauba wax, rose wax as a thickening agent; ceteareth-30, PEG-40, hydrogenated castor oil, lanolin as an emulsifier; propylene glycol, ethoxydiglycol as a solvent; cetearylmethicone, dimethiconol as conditioning agents; polyquaternium-16, polyquaternium-7 as cationic polymers; quaternium-70, becentrimonium chloride as cationic surfactants; cocamide MEA as a nonionic surfactant; sodium sulfite, sodium erythorbate, sodium hydrosulfite as reducing agents; tetrasodium salt of ethylenediaminetetraacetic acid as a water-softening complexone; methylparaben, ethylparaben, propylparaben in phenoxyethanol as preservative agents; perfume composition and water at the following proportions of components in the specified emulsion; in % by weight:

| | |
|---|---|
| thickening agent: carnauba wax, rose wax | 0.6-5.5; |
| emulsifier: | |
| ceteareth-30, lanolin, PEG-40, hydrogenated castor oil | 2.0-24.0; |
| organic solvent: propylene glycol, ethoxydiglycol | 1.5-14.0; |
| coloring component | 0.0001-10.0; |
| conditioning agent: cetearylmethicone, dimethiconol | 1.0-10.0; |
| cationic polymer: polyquaternium-16, polyquaternium-7 | 1.0-9.0; |
| cationic surfactant: quaternium-70, becentrimonium chloride | 2.0-30.0; |
| non-ionic surfactant: cocamide MEA | 1.0-15.0; |
| reducing agent: sodium sulfite, sodium erythorbate, sodium hydrogen sulphite | 0.31-3.5; |
| perfume composition | 0.1-1.2; |
| preservative agent: methylparaben, propylparaben, ethylparaben in phenoxyethanol | 0.1-1.0; |
| complexone: | |
| tetrasodium salt of ethylenediaminetetraacetic acid | 0.1-1.0; |
| mica, titanium dioxide | 0.1-1.0; |
| water | up to 100. |

Additional experiment data

Table 9 shows the data on mechanical testing of hair treated with coloring formulations without the composition of the invention (samples 3[0], 5[0]), coloring formulations with addition of the composition of the invention containing 1,2-ethylenediamine dihydrochloride in a concentration of 10% (samples 3[3], 5[3]) and 20% (samples 3[4], 5[4]); 1,2-ethylenediamine dihydrobromide in a concentration of 10% (samples 3[5], 5[5]) and 20% (samples 3[6], 5[6]); 1,2-ethylenediamine sulfate in a concentration of 10% (samples 3[7], 5[7]) and 20% (samples 3[8], 5[8]), calculated with a confidence coefficient of 95%.

14

Table 9. Deformation-strength properties of human hair samples after treatment with a formulation containing salts of 1,2-ethylenediamine.

| Sample No. | Average diameter, mm | Yield strength, MPa | Breaking strength, MPa | Breaking elongation, % | Initial tangent modulus of elasticity, GPa |
|---|---|---|---|---|---|
| $3^0$ | 0.057 | 248 ± 23 | 419 ± 36 | 49 ± 3 | 8.0 ± 0.8 |
| $3^3$ | 0.056 | 249 ± 22 | 418 ± 34 | 47 ± 3 | 9.1 ± 1.0 |
| $3^4$ | 0.055 | 258 ± 20 | 418 ± 37 | 48 ± 3 | 9.8 ± 1.0 |
| $3^3$ | 0.056 | 248 ± 21 | 420 ± 34 | 49 ± 2 | 9.6 ± 1.2 |
| $3^6$ | 0.056 | 250 ± 22 | 421 ± 37 | 48 ± 2 | 9.9 ± 1.0 |
| $3^7$ | 0.057 | 252 ± 20 | 422 ± 35 | 47 ± 2 | 9.9 ± 0.8 |
| $3^8$ | 0.056 | 256 ± 18 | 422 ± 31 | 46 ± 1 | 9.9 ± 0.6 |
| $5^0$ | 0.061 | 194 ± 17 | 329 ± 31 | 46 ± 4 | 6.6 ± 0.6 |
| $5^3$ | 0.060 | 226 ± 17 | 376 ± 32 | 49 ± 4 | 9.5 ± 1.0 |
| $5^4$ | 0.062 | 235 ± 21 | 380 ± 31 | 48 ± 4 | 9.9 ± 0.8 |
| $5^5$ | 0.062 | 222 ± 21 | 375 ± 30 | 47 ± 3 | 9.8 ± 1.0 |
| $5^6$ | 0.059 | 228 ± 21 | 377 ± 31 | 49 ± 2 | 9.9 ± 1.0 |
| $5^7$ | 0.060 | 216 ± 31 | 352 ± 31 | 46 ± 2 | 9.5 ± 1.4 |
| $5^8$ | 0.061 | 221 ± 29 | 360 ± 30 | 45 ± 3 | 9.7 ± 1.4 |

Table 10 shows the data on mechanical testing of hair treated with coloring formulations without the composition of the invention (samples $6^0$, $9^0$), coloring formulations with addition of the composition of the invention containing 1,6-hexanediamine dihydrochloride in a concentration of 10% (samples $6^3$, $9^3$) and 20% (samples $6^4$, $9^4$); 1,6-hexanediamine dihydrobromide in a concentration of 10% (samples $6^5$, $9^5$) and 20% (samples $6^6$, $9^6$); 1,6-hexanediamine sulfate in a concentration of 10% (samples $6^7$, $9^7$) and 20% (samples $6^8$, $9^8$), calculated with a confidence coefficient of 95%.

Table 10. Deformation-strength properties of human hair samples after treatment with a formulation containing salts of 1,6-hexanediamine.

| Sample No. | Average diameter, mm | Yield strength, MPa | Breaking strength, MPa | Breaking elongation, % | Initial tangent modulus of elasticity, GPa |
|---|---|---|---|---|---|
| $6^0$ | 0.056 | 204 ± 19 | 357 ± 34 | 49 ± 4 | 6.8 ± 0.8 |
| $6^3$ | 0.055 | 220 ± 23 | 370 ± 47 | 49 ± 3 | 9.0 ± 1.0 |
| $6^4$ | 0.056 | 231 ± 21 | 393 ± 50 | 49 ± 4 | 9.6 ± 1.0 |
| $6^5$ | 0.054 | 222 ± 21 | 372 ± 39 | 48 ± 3 | 9.1 ± 1.0 |
| $6^6$ | 0.055 | 231 ± 24 | 386 ± 46 | 49 ± 4 | 9.5 ± 1.3 |
| $6^7$ | 0.055 | 221 ± 23 | 371 ± 42 | 47 ± 4 | 9.0 ± 0.9 |
| $6^8$ | 0.057 | 227 ± 22 | 389 ± 52 | 49 ± 3 | 9.6 ± 1.2 |
| $9^0$ | 0.057 | 245 ± 22 | 424 ± 31 | 51 ± 4 | 8 ± 0.8 |
| $9^3$ | 0.057 | 274 ± 30 | 444 ± 53 | 42 ± 4 | 11.0 ± 1.1 |
| $9^4$ | 0.058 | 296 ± 27 | 458 ± 51 | 47 ± 1.5 | 11.4 ± 1.0 |
| $9^5$ | 0.058 | 284 ± 28 | 440 ± 51 | 41 ± 4 | 11.1 ± 1.0 |
| $9^6$ | 0.057 | 290 ± 31 | 460 ± 52 | 42 ± 3 | 10.8 ± 1.1 |
| $9^7$ | 0.056 | 276 ± 24 | 451 ± 50 | 43 ± 3 | 10.9 ± 0.9 |
| $9^8$ | 0.058 | 289 ± 31 | 459 ± 49 | 43 ± 2 | 11.0 ± 0.9 |

Table 11 shows the data on mechanical testing of hair treated with coloring formulations without the composition of the invention (samples $3^0$, $5^0$), coloring formulations with addition of the composition of the invention containing 1,3-propylenediamine in a concentration of 10% (samples $3^9$, $5^9$) and 20% (samples $3^{10}$, $5^{10}$); 1,3-propylenediamine dihydrochloride in a concentration of 10% (samples $3^{11}$, $5^{11}$) and 20% (samples $3^{12}$, $5^{12}$); 1,3-propylenediamine dihydrobromide in a concentration of 10% (samples $3^{13}$, $5^{13}$) and 20% (samples $3^{14}$, $5^{14}$); 1,3-propylenediamine sulfate in a concentration of 10% (samples $3^{15}$, $5^{15}$) and 20% (samples $3^{16}$, $5^{16}$), calculated with a confidence coefficient of 95%.

Table 11. Deformation-strength properties of human hair samples after treatment with a formulation containing 1,3-propylenediamine and its salts.

| Sample No. | Average diameter, mm | Yield strength, MPa | Breaking strength, MPa | Breaking elongation, % | Initial tangent modulus of elasticity, GPa |
|---|---|---|---|---|---|
| $3^0$ | 0.057 | 248 ± 23 | 419 ± 36 | 49 ± 3 | 8.0 ± 0.8 |
| $3^9$ | 0.056 | 251 ± 22 | 420 ± 38 | 48 ± 2 | 9.0 ± 1.1 |
| $3^{10}$ | 0.056 | 263 ± 21 | 429 ± 37 | 47 ± 3 | 9.7 ± 0.8 |
| $3^{11}$ | 0.057 | 253 ± 22 | 422 ± 34 | 46 ± 3 | 9.2 ± 1.2 |
| $3^{12}$ | 0.057 | 259 ± 20 | 428 ± 38 | 48 ± 2 | 9.8 ± 0.8 |
| $3^{13}$ | 0.055 | 253 ± 20 | 420 ± 37 | 46 ± 2 | 9.2 ± 1.0 |
| $3^{14}$ | 0.057 | 261 ± 23 | 431 ± 35 | 47 ± 3 | 9.6 ± 1.0 |
| $3^{15}$ | 0.056 | 255 ± 23 | 426 ± 38 | 47 ± 2 | 9.1 ± 1.1 |
| $3^{16}$ | 0.058 | 263 ± 22 | 444 ± 37 | 46 ± 2 | 9.8 ± 1.2 |
| $5^0$ | 0.061 | 194 ± 17 | 329 ± 31 | 46 ± 4 | 6.6 ± 0.6 |
| $5^9$ | 0.062 | 222 ± 26 | 365 ± 30 | 45 ± 2 | 9.6 ± 1.2 |
| $5^{10}$ | 0.061 | 239 ± 21 | 375 ± 32 | 44 ± 4 | 9.8 ± 1.1 |
| $5^{11}$ | 0.059 | 223 ± 20 | 364 ± 33 | 45 ± 3 | 9.3 ± 1.0 |
| $5^{12}$ | 0.059 | 237 ± 23 | 379 ± 32 | 45 ± 2 | 9.8 ± 0.8 |
| $5^{13}$ | 0.060 | 231 ± 21 | 360 ± 32 | 44 ± 3 | 9.3 ± 0.9 |
| $5^{14}$ | 0.059 | 240 ± 22 | 371 ± 33 | 44 ± 2 | 9.9 ± 1.0 |
| $5^{15}$ | 0.062 | 227 ± 21 | 358 ± 30 | 43 ± 4 | 9.0 ± 1.6 |
| $5^{16}$ | 0.061 | 234 ± 20 | 365 ± 31 | 45 ± 3 | 9.7 ± 1.1 |

Table 12 shows the data on mechanical testing of hair treated with coloring formulations without the composition of the invention (samples $3^0$, $5^0$), coloring formulations with addition of the composition of the invention containing 1,4-butanediamine in a concentration of 10% (samples $3^{17}$, $5^{17}$); 1,4-butanediamine dihydrochloride in a concentration of 10% (samples $3^{18}$, $5^{18}$); 1,4-butanediamine dihydrobromide in a concentration of 10% (samples $3^{19}$, $5^{19}$); 1,4-butanediamine sulfate in a concentration of 10% (samples $3^{20}$, $5^{20}$); 1,5-pentanediamine in a concentration of 10% (samples $3^{21}$, $5^{21}$); 1,5-pentanediamine dihydrochloride in a concentration of 10% (samples $3^{22}$, $5^{22}$); 1,5-pentanediamine dihydrobromide in a concentration of 10% (samples $3^{23}$, $5^{23}$); 1,5-pentanediamine sulfate in a concentration of 10% (samples $3^{24}$, $5^{24}$), calculated with a confidence coefficient of 95%.

Table 12. Deformation-strength properties of human hair samples after treatment with a formulation containing 1,4-butanediamine and its salts and 1,5-pentanediamine and its salts.

| Sample No. | Average diameter, mm | Yield strength, MPa | Breaking strength, MPa | Breaking elongation, % | Initial tangent modulus of elasticity, GPa |
|---|---|---|---|---|---|
| $3^0$ | 0.057 | 248 ± 23 | 419 ± 36 | 48 ± 3 | 8.0 ± 0.8 |
| $3^{17}$ | 0.057 | 249 ± 21 | 420 ± 38 | 48 ± 3 | 9.1 ± 1.0 |
| $3^{18}$ | 0.055 | 252 ± 22 | 419 ± 36 | 47 ± 2 | 9.1 ± 0.8 |

(continued)

| Sample No. | Average diameter, mm | Yield strength, MPa | Breaking strength, MPa | Breaking elongation, % | Initial tangent modulus of elasticity, GPa |
|---|---|---|---|---|---|
| 3[19] | 0.056 | 250 ± 22 | 420 ± 35 | 47 ± 3 | 9.2 ± 1.1 |
| 3[20] | 0.056 | 249 ± 23 | 421 ± 38 | 48 ± 2 | 9.0 ± 0.9 |
| 3[21] | 0.057 | 251 ± 21 | 419 ± 37 | 48 ± 3 | 9.1 ± 1.0 |
| 3[22] | 0.056 | 249 ± 23 | 422 ± 37 | 47 ± 3 | 9.0 ± 1.1 |
| 3[23] | 0.057 | 251 ± 21 | 421 ± 36 | 47 ± 3 | 9.1 ± 1.2 |
| 3[24] | 0.056 | 253 ± 22 | 420 ± 39 | 48 ± 2 | 9.3 ± 0.8 |
| 5[0] | 0.061 | 194 ± 17 | 329 ± 31 | 46 ± 4 | 6.6 ± 0.6 |
| 5[17] | 0.060 | 199 ± 24 | 340 ± 31 | 45 ± 3 | 8.6 ± 1.0 |
| 5[18] | 0.062 | 201 ± 23 | 342 ± 33 | 45 ± 4 | 8.7 ± 1.1 |
| 5[19] | 0.061 | 203 ± 20 | 341 ± 34 | 44 ± 3 | 9.0 ± 1.0 |
| 5[20] | 0.060 | 198 ± 21 | 341 ± 36 | 46 ± 2 | 8.8 ± 0.8 |
| 5[21] | 0.062 | 201 ± 22 | 340 ± 33 | 46 ± 2 | 8.9 ± 1.2 |
| 5[22] | 0.061 | 200 ± 21 | 343 ± 32 | 45 ± 3 | 8.9 ± 1.0 |
| 5[23] | 0.060 | 197 ± 23 | 340 ± 30 | 46 ± 3 | 9.0 ± 0.6 |
| 5[24] | 0.062 | 202 ± 23 | 345 ± 32 | 44 ± 2 | 9.0 ± 1.0 |

As it seen from the experimental data presented above, use of coloring formulations without the composition of the invention results in a decrease in the yield strength, breaking strength and initial tangent modulus of elasticity on average from 15 to 30%. In case of coloring the samples with coloring formulations with $\alpha,\omega$-diamines of the invention, with increase of their concentration, an improvement in the yield strength, breaking strength and tangent modulus of elasticity in comparison with human hair colored with control dye occurs. A subject matter expert can unambiguously conclude that introduction of $\alpha,\omega$-diamines salts of the invention into the coloring formulation improves the key parameters of the hair mechanical strength. The results are very similar to the data obtained with use of individual $\alpha,\omega$-diamines, which unambiguously allows to confirm that improvement of the hair strength occurs due to molecules of $\alpha,\omega$-diamines of the invention and their cationic (salt) forms.

The effect of concentration of the specified $\alpha,\omega$-diamines on achievement of the expected technical result and possibility of practical application of compositions with $\alpha,\omega$-diamines in various concentrations was also studied. Below is the data for concentrations of 1% and 96%. It should again be noted that the maximum concentration in commercial hair care products may be limited not by a certain change (loss) of the ability to improve the hair mechanical strength, but by limits in which the technological properties of the component system must fall, for example, by acceptable solubility, etc.

Table 13 shows the data on mechanical testing of hair treated with coloring formulations without the composition of the invention (samples 5[0], 6[0]), coloring formulations with addition of the composition of the invention containing 1,2-ethylenediamine in a concentration of 1% (sample 5[a1]) and 96% (sample 5[a2]); 1,6-hexamethylenediamine in a concentration of 1% (sample 6[b1]) and 96% (sample 6[b2]), calculated with a confidence coefficient of 95%.

Table 13. Study of minimum and maximum concentrations of certain $\alpha,\omega$-diamines on hair mechanical properties.

| Sample No. | Average diameter, mm | Yield strength, MPa | Breaking strength, MPa | Breaking elongation, % | Initial tangent modulus of elasticity, GPa |
|---|---|---|---|---|---|
| 5[0] | 0.061 | 194 ± 17 | 329 ± 31 | 46 ± 4 | 6.6 ± 0.6 |
| 5[a1] | 0.056 | 194 ± 22 | 330 ± 32 | 47 ± 2 | 7.0 ± 1.0 |
| 5[a2] | 0.060 | 230 ± 23 | 373 ± 35 | 48 ± 2 | 9.9 ± 1.1 |

(continued)

| Sample No. | Average diameter, mm | Yield strength, MPa | Breaking strength, MPa | Breaking elongation, % | Initial tangent modulus of elasticity, GPa |
|---|---|---|---|---|---|
| $6^0$ | 0.056 | $204 \pm 19$ | $357 \pm 34$ | $49 \pm 4$ | $6.8 \pm 0.8$ |
| $6^{b1}$ | 0.061 | $204 \pm 20$ | $358 \pm 33$ | $48 \pm 3$ | $6.9 \pm 1.0$ |
| $6^{b2}$ | 0.060 | $231 \pm 22$ | $384 \pm 39$ | $47 \pm 2$ | $10.0 \pm 0.8$ |

The presented examples do not limit the present invention, but serve only to illustrate its embodiments.

**Claims**

1.  Hair care product containing at least one $\alpha,\omega$-diamine of the general formula $H_2N\text{-}CH_2\text{-}(CH_2)n\text{-}CH_2\text{-}NH_2$ or its salt, where n = 0, 1, 2, 3, 4 or 5, which improves the hair mechanical strength.

2.  Hair care product according to item 1, where at least one specified $\alpha,\omega$-diamine is 1,2-ethylenediamine, 1,3-propylenediamine, 1,4-butanediamine, 1,5-pentanediamine, 1,6-hexanediamine or 1,7- heptanediamine.

3.  Hair care product according to item 2, where at least one specified $\alpha,\omega$-diamine is 1,2-ethylenediamine, 1,3-propylenediamine or 1,6-hexanediamine.

4.  Hair care product according to item 1, where at least one salt of at least one specified $\alpha,\omega$-diamine is a salt of sulfuric, hydrochloric, phosphoric, lactic, citric or acetic acids.

5.  Hair care product according to item 1, where the specified product is a single-phase or multi-phase coloring formulation, perming formulation, straightening formulation, essence, balm, shampoo, conditioner, rinse, mask, tonic, lotion, milk, emulsion, gel, oil, gloss, wax, clay, paste, serum, cream, spray, mousse, foam or fluid.

6.  Hair care product according to item 5, where the specified product is a multi-phase coloring formulation consisting of Phase A, Phase B and Phase C separated from each other and mixed immediately before use, where:

    - Phase A contains alkaline agents, thickeners in the form of associated polymers, non-associated cross-linked polymers, polysaccharides, polycarboxylates, polyacrylates, or derivatives, or mixtures thereof; waxes, essential and mineral oils; cationic, anionic, non-ionic surfactants; cationic and anionic polymers; reducing agents in the form of inorganic salts; emulsifiers in the form of fatty alcohols, fatty esters, fatty acids; organic solvents in the form of mono- and polyatomic alcohols; silicones, preservative agents, moisturizing agents, emollients, complexones, fragrances, oxidation coloring components, color components such as m-aminophenols, di- and trihydroxy derivatives of the aromatic and heterocyclic series, nitro-, chloro-, methoxy- and ethoxy derivatives thereof; direct dyes, such as acidic, basic, cationic, active, dispersed, vat dyes, which are nitric, anthraquinone, azo, arylmethane, arylamine, phthalocyanine, indigoid, and thioindigoid dyes, and their heterocyclic analogues; water;
    - Phase B contains hydrogen peroxide, thickeners, emulsifiers, emollients, surfactants, complexones;
    - Phase C contains at least one specified $\alpha,\omega$-diamine, cationic surfactants, moisturizing agents, silicones, thickeners, preservative agents, water.

7.  Hair care product according to item 1, where at least one specified $\alpha,\omega$-diamine or its salt may be present in an amount of about 1-50% by weight, preferentially about 3-40% by weight, more preferentially about 5-30% by weight, most preferentially about 8-20% by weight.

8.  Hair care product according to item 1, which additionally contains cetrimonium chloride, panthenol, quaternium-18 silicone emulsion with trideceth-6 and trideceth-12, hydroxyethylcellulose, preservative agent, water.

9.  Hair care product according to item 8, which contains, in % by weight:

| | |
|---|---|
| specified $\alpha,\omega$-diamine | from about 8.0 to about 20.0; |
| cetrimonium chloride | from about 0.5 to about 10.0; |
| panthenol | from about 1.5 to about 10.0; |
| quaternium-18 silicone emulsion with trideceth-6 and trideceth-12 | from about 1.5 to about 10.0; |
| hydroxyethylcellulose | from about 0.05 to about 1.0; |
| preservative agent | from about 0.01 to about 1.0; |
| water | up to 100. |

10. Application of at least one $\alpha,\omega$-diamine of the general formula $H_2N\text{-}CH_2\text{-}(CH_2)_n\text{-}CH_2\text{-}NH_2$ or its salt, where n = 0, 1, 2, 3, 4 or 5, in a hair care product according to any of items 1-9 to improve the hair mechanical strength.

11. Application according to item 10, where at least one specified $\alpha,\omega$-diamine is 1,2-ethylenediamine, 1,3-propylenediamine, 1,4-butanediamine, 1,5-pentanediamine, 1,6-hexanediamine or 1,7- heptanediamine.

12. Application according to item 11, where at least one specified $\alpha,\omega$-diamine is 1,2-ethylenediamine, 1,3-propylenediamine or 1,6-hexanediamine.

13. Application according to item 10, where at least one salt of at least one specified $\alpha,\omega$-diamine is a salt of sulfuric, hydrochloric, phosphoric, lactic, citric or acetic acids.

14. Application of at least one $\alpha,\omega$-diamine of the general formula H2N-CH2-(CH2)n-CH2-NH2 or its salt, where n = 0, 1, 2, 3, 4 or 5, to improve the hair mechanical strength.

15. Application according to item 14, where at least one specified $\alpha,\omega$-diamine is 1,2-ethylenediamine, 1,3-propylenediamine, 1,4-butanediamine, 1,5-pentanediamine, 1,6-hexanediamine or 1,7- heptanediamine.

16. Application according to item 15, where at least one specified $\alpha,\omega$-diamine is 1,2-ethylenediamine, 1,3-propylenediamine or 1,6-hexanediamine.

17. Application according to item 14, where at least one salt of at least one specified $\alpha,\omega$-diamine is a salt of sulfuric, hydrochloric, phosphoric, lactic, citric or acetic acids.

## INTERNATIONAL SEARCH REPORT

| International application No |
| --- |
| PCT/RU2018/050111 |

**A. CLASSIFICATION OF SUBJECT MATTER**
INV. A61K8/44    A61Q5/00
ADD.

According to International Patent Classification (IPC) or to both national classification and IPC

**B. FIELDS SEARCHED**

Minimum documentation searched (classification system followed by classification symbols)
A61K  A61Q

Documentation searched other than minimum documentation to the extent that such documents are included in the fields searched

Electronic data base consulted during the international search (name of data base and, where practicable, search terms used)

EPO-Internal, BIOSIS, CHEM ABS Data, COMPENDEX, EMBASE, EMBL, FSTA, INSPEC, IBM-TDB, WPI Data

**C. DOCUMENTS CONSIDERED TO BE RELEVANT**

| Category* | Citation of document, with indication, where appropriate, of the relevant passages | Relevant to claim No. |
| --- | --- | --- |
| X | DATABASE GNPD [Online]<br>MINTEL;<br>1 October 2001 (2001-10-01),<br>anonymous: "Haircare Range",<br>XP055544031,<br>retrieved from www.gnpd.com<br>Database accession no. 115372<br>the whole document<br>----- | 1-5,<br>10-13 |
| X | DATABASE GNPD [Online]<br>MINTEL;<br>13 October 2005 (2005-10-13),<br>anonymous: "Ultra Strong Styling Gel",<br>XP055544045,<br>retrieved from www.gnpd.com<br>Database accession no. 404875<br>the whole document<br>----- | 1-5,<br>10-13 |

-/--

[X] Further documents are listed in the continuation of Box C.       [X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 22 January 2019 | 12/02/2019 |

| Name and mailing address of the ISA/<br>European Patent Office, P.B. 5818 Patentlaan 2<br>NL - 2280 HV Rijswijk<br>Tel. (+31-70) 340-2040,<br>Fax: (+31-70) 340-3016 | Authorized officer<br><br>Irwin, Lucy |

Form PCT/ISA/210 (second sheet) (April 2005)

## INTERNATIONAL SEARCH REPORT

| | International application No |
|---|---|
| | PCT/RU2018/050111 |

C(Continuation). DOCUMENTS CONSIDERED TO BE RELEVANT

| Category* | Citation of document, with indication, where appropriate, of the relevant passages | Relevant to claim No. |
|---|---|---|
| X | DATABASE GNPD [Online]<br>MINTEL;<br>8 March 2012 (2012-03-08),<br>anonymous: "Hair Styling Gel",<br>XP055544062,<br>retrieved from www.gnpd.com<br>Database accession no. 1745406<br>abstract<br>----- | 1-3,5,<br>10-12 |
| X | GB 1 186 101 A (OREAL [FR])<br>2 April 1970 (1970-04-02)<br>example 7<br>----- | 1-3,5,<br>10-12 |
| X | WO 2013/098332 A2 (OREAL [FR])<br>4 July 2013 (2013-07-04)<br>examples 1,2<br>----- | 1-3,5,<br>10-12 |
| X | WO 00/71658 A1 (PROCTER & GAMBLE [US];<br>KASTURI CHANDRIKA [US]; SCHAFER MICHAEL<br>GAYLE []  30 November 2000 (2000-11-30)<br>examples 35,37,39<br>----- | 1-3,5,<br>10-12 |
| A | GB 2 386 068 A (PROCTER & GAMBLE [US])<br>10 September 2003 (2003-09-10)<br>the whole document<br>----- | 1-17 |
| A | FR 1 146 332 A (CHARRET)<br>8 November 1957 (1957-11-08)<br>page 1<br>claim 1<br>----- | 1-17 |
| A | US 2 930 761 A (CHARRET EDOUARD J-F)<br>29 March 1960 (1960-03-29)<br>page 1<br>claim 1<br>----- | 1-17 |

Form PCT/ISA/210 (continuation of second sheet) (April 2005)

## INTERNATIONAL SEARCH REPORT
Information on patent family members

International application No

PCT/RU2018/050111

| Patent document cited in search report | | Publication date | Patent family member(s) | | Publication date |
|---|---|---|---|---|---|
| GB 1186101 | A | 02-04-1970 | BE | 694956 A | 04-09-1967 |
| | | | CH | 471582 A | 30-04-1969 |
| | | | DE | 1617710 A1 | 25-03-1971 |
| | | | GB | 1186101 A | 02-04-1970 |
| | | | GB | 1186102 A | 02-04-1970 |
| | | | IT | 988301 B | 10-04-1975 |
| | | | LU | 50572 A1 | 05-09-1967 |
| | | | NL | 6703296 A | 05-09-1967 |
| WO 2013098332 | A2 | 04-07-2013 | NONE | | |
| WO 0071658 | A1 | 30-11-2000 | AR | 020297 A1 | 02-05-2002 |
| | | | AU | 5444100 A | 12-12-2000 |
| | | | BR | 0011549 A | 26-02-2002 |
| | | | CA | 2372894 A1 | 30-11-2000 |
| | | | CA | 2688927 A1 | 30-11-2000 |
| | | | EP | 1180132 A1 | 20-02-2002 |
| | | | JP | 2003500525 A | 07-01-2003 |
| | | | MX | PA01012189 A | 30-07-2002 |
| | | | WO | 0071658 A1 | 30-11-2000 |
| GB 2386068 | A | 10-09-2003 | AT | 454880 T | 15-01-2010 |
| | | | AU | 2002252417 B2 | 12-05-2005 |
| | | | CA | 2437051 A1 | 26-09-2002 |
| | | | CN | 1496247 A | 12-05-2004 |
| | | | DK | 1370222 T3 | 26-04-2010 |
| | | | EP | 1370222 A1 | 17-12-2003 |
| | | | ES | 2338301 T3 | 06-05-2010 |
| | | | GB | 2386068 A | 10-09-2003 |
| | | | JP | 3995599 B2 | 24-10-2007 |
| | | | JP | 2004524332 A | 12-08-2004 |
| | | | MX | PA03008246 A | 29-01-2004 |
| | | | WO | 02074272 A1 | 26-09-2002 |
| FR 1146332 | A | 08-11-1957 | CH | 357152 A | 30-09-1961 |
| | | | DE | 1083504 B | 15-06-1960 |
| | | | DK | 88850 C | 19-04-1960 |
| | | | FR | 1146332 A | 08-11-1957 |
| | | | GB | 823303 A | 11-11-1959 |
| | | | NL | 98239 C | 15-06-1961 |
| | | | US | 2930761 A | 29-03-1960 |
| US 2930761 | A | 29-03-1960 | CH | 357152 A | 30-09-1961 |
| | | | DE | 1083504 B | 15-06-1960 |
| | | | DK | 88850 C | 19-04-1960 |
| | | | FR | 1146332 A | 08-11-1957 |
| | | | GB | 823303 A | 11-11-1959 |
| | | | NL | 98239 C | 15-06-1961 |
| | | | US | 2930761 A | 29-03-1960 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7198651 B2
- US 7241318 B2
- US 6740129 B2
- US 7204856 B2
- US 7300470 B2
- US 7204859 B2
- WO 2009077302 A2
- DE 102006055436 A1
- FR 2870733 B1
- US 7044986 B2
- US 7056497 B2
- RU 2493814 C1
- RU 2541811 C1
- RU 2572708 C2
- RU 2602690 C1
- RU 2623033 C1
- RU 2452466 C2
- US 7211243 B2